# EUROPEAN PATENT SPECIFICATION

(11) **EP 2 358 708 B1**
(45) Date of publication and mention of the grant of the patent: **12.06.2013**
(21) Application number: 08878694.2
(22) Date of filing: 08.12.2008
(51) Int. Cl.: C07D 411/04

(54) **OPTICAL RESOLUTION OF SUBSTITUTED 1.3-OXATHIOLANE NUCLEOSIDES**
OPTISCHE AUFLÖSUNG SUBSTITUIERTER 1,3-OXATHIOLAN-NUKLEOSIDE
RÉSOLUTION OPTIQUE DE NUCLÉOSIDES 1,3-OXATHIOLANE SUBSTITUÉS

(43) Date of publication of application: 24.08.2011
(73) Proprietor: Hetero Research Foundation, Hyderabad 500 018 (IN)
(72) Inventor: PARTHASARADHI REDDY, Bandi, 500018 Hyderabad (IN); RATHNAKAR REDDY, Kura, 500018 Hyderabad (IN); RAJI REDDY, Rapolu, 500018 Hyderabad (IN); MURALIDHARA REDDY, Dasari, 500018 Hyderabad (IN); SATYA SRINIVAS, Ayyalsomayajula, 500018 Hyderabad (IN)
(74) Representative: Thomas, Simon
(86) International application number: PCT/IN2008/000823
(87) International publication number: WO 2010/067375

(56) References cited:
- WO-A1-2006/096954
- WO-A1-2006/096954
- WO-A2-2008/053496
- "Resolving Agents for Bases" In: Eliel, E.L.; Wilen, S.H.; Mander, L.N.: "Stereochemistry of Organic Compounds", 1994, John Wiley & Sons, New York, XP002674848, ISBN: 0-471-01670-5 pages 332-335, * Paragraph Bridging pages 332 and 333; page 332 - page 335; compound 65 *

## Description

### FIELD OF THE INVENTION

The present invention relates to process for optical resolution of substituted 1,3-oxathiolane nucleosides.

### BACKGROUND OF THE INVENTION

Antiretroviral activity of 2-substitued-5-substitued-1,3-oxathiolanes were disclosed in US Patent No. 5,047,407. Of the compounds, Lamivudine, chemically (2R-cis)-4-amino-1-[2-(hydroxymethyl)-1,3-oxathiolan-5-yl]-2(1H)-pyrimidinone is marketed under the brand EPIVIR. Lamivudine is represented by the following structure of formula (I).

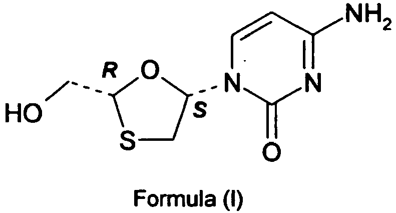

(2R-cis)-4-Amino-1-[2-(hydroxymethyl)-1,3-oxathiolan-5-yl]-2(1H)-pyrimidinone is also useful in the treatment of hepatitis B infection as disclosed in US RE39155. WO Patent Publication No. 92/20344 disclosed a method of treatment of HIV infection and other viral infection with lamivudine in combination with other antiviral agents such as Zidovudine, chemically 3'-azido-3'-deoxythymidine.

Lamivudine may be prepared using the procedures described in US Patent No. 5,047,407. US Patent No. 5,047,407 disclosed the 1,3-oxathiolane derivatives; their geometric (cis/trans) and optical isomers. This patent described the preparation of 2-substitued-5-substitued-1,3-oxathiolanes. US Patent No. 5,047,407 discribed the preparation of invidual stereoisomers of 2-substitued-5-substitued-1,3-oxathiolanes from stereoisomerically pure raw materials or intermediates.

US Patent No. 5,248,776 described an asymmetric process for the synthesis of enantiomerically pure β-L-(-)-1,3-oxathiolane-nucleosides starting from optically pure 1,6-thioanhydro-L-gulose to give first the [2R,5R] and [2R, 5S] diastereomers that are then separated chromatographically.

US Patent No. 6,600,044 described a method for converting the undesired trans-1,3-oxathiolane nucleoside to the desired cis isomer by a method of anomerization or transglycosylation and the separation of the hydroxyl-protected form of cis-, trans-(-)-nucleosides by fractional crystallization of their hydrochloride, hydrobromide, methanesulfonate salts.

WO Patent Publication No. 2008/053496 disclosed a process for the resolution of cis(±)-4-amino-1-[2-(hydroxymethyl)-1,3-oxathiolan-5-yl]-2(1H)-pyrimidinone by using (S)-BINOL. We have found that the process is not reproducible.

Eliel EL, Wilen SH, Mander LN Stereochemistry of Organic Compounds, 1994, John Wiley & Sons, New York, pp 332-335 discloses that (S)-(+)-1,1'-binaphthyl-2,2'-diyl hydrogen phosphate can be useful as a resolving agent, in particular for the resolution of tertiary amines.

WO 2006/096954 discloses a process for the preparation of optically-active cis-2-hydroxymethyl-4-(cytosine-1'-yl)-1,3-oxathiolane or its pharmaceutically acceptable salts.

We have found that the use of various chiral acids such as chiral camphorsulfonic acid, camphanic acid, tartaric acid, ditoluoyl tartaric acid, dibenzoyl tartaric acid, proline and malic acid for the resolution of racemic-4-amino-1-[2-(hydroxymethyl)-1,3-oxathiolan-5-yl]-2(1 H)-pyrimidinone does not result in the separation of enantiomers.

We have discovered a novel process for optical resolution of cis(±)-4-amino-1-[2-(hydroxymethyl)-1,3-oxathiolan-5-yl]-2(1H)-pyrimidinone and trans(±)-4-amino-1-[2-(hydroxymethyl)-1,3-oxathiolan-5-yl]-2(1H)-pyrimidinone. The object of the present invention is to provide an improved and commercially viable process for optical resolution of cis(±)-4-amino-1-[2-(hydroxymethyl)-1,3-oxathiolan-5-yl]-2(1H)-pyrimidinone and trans(±)-4-amino-1-[2-(hydroxymethyl)-1,3-oxathiolan-5-yl]-2(1H)-pyrimidinone. The process of the invention can be applied to obtain an enantiomer of (2R-cis)-4-amino-1-[2-(hydroxymethyl)-1 ,3-oxathiolan-5-yl]-2(1 H)-pyrimidinone with an optical purity to the extent of 100%.

### SUMMARY OF THE INVENTION

According to one aspect of the present invention, there is provided a process for resolution of ds(±)-4-amino-1-[2-(hydroxymethyl)-1,3-oxathiolan-5-yl]-2(1H)-pyrimidinone or trans(±)-4-amino-1-[2-(hydroxymethyl)-1,3-oxathiolan-5-yl]-2(1H)-pyrimidinone; or a mixture thereof which comprises:
a) reacting cis(±)-4-amino-1-[2-(hydroxymethyl)-1,3-oxathiolan-5-yl]-2(1H)-pyrimidinone or trans(±)-4-amino-1-[2-(hydroxymethyl)-1,3-oxathiolan-5-yl]-2(1H)-pyrimidinone; or a mixture thereof with a chiral 1,1'-binaphtyl-2-2'-diyl hydrogen phosphate (BNPPA) to obtain corresponding one or more diastereomer compounds;
b) selectively crystallizing one diastereomer compound from water or water in combination with a solvent at a pH of 8.0 to 12.0; and
c) converting the separated diastereomer or diastereomers to the corresponding (2R-cis)-4-amino-1-[2-(hydroxymethyl)-1,3-oxathiolan-5-yl]-2(1H)-pyrimidinone, (2S-cis)-4-amino-1-[2-(hydroxymethyl)-1,3-oxathiolan-5-yl]-2(1H)-pyrimidinone, (2S-trans)-4-amino-1-[2-(hydroxymethyl)-1,3-oxathiolan-5-yl]-2(1H)-pyrimidinone or (2R-trans)-4-amino-1-[2-(hydroxymethyl)-1,3-oxathiolan-5-yl]-2(1H)-pyrimidinone.

According to another aspect of the present invention there is provided a novel compounds of formula IIa to IIh.

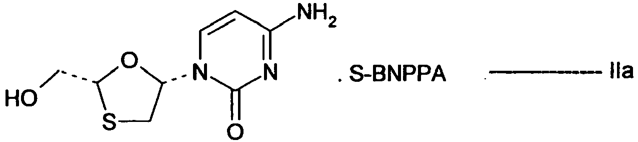

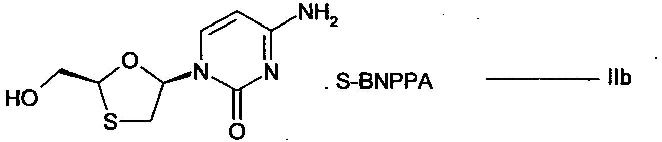

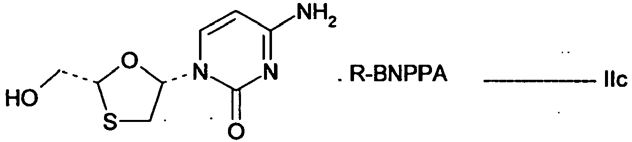

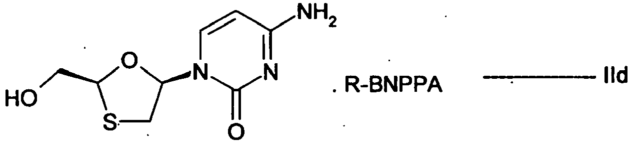

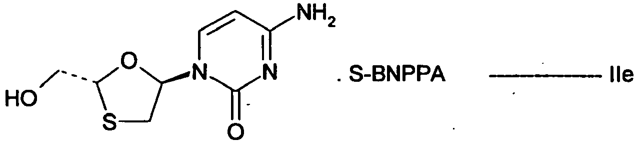

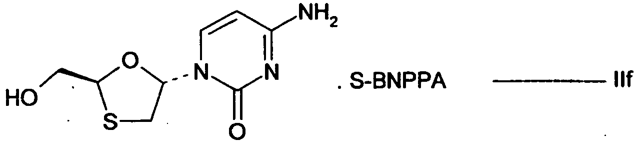

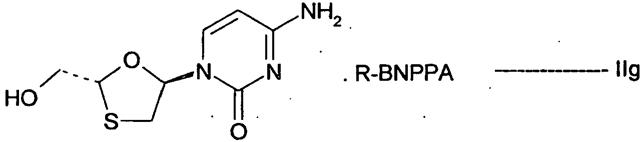

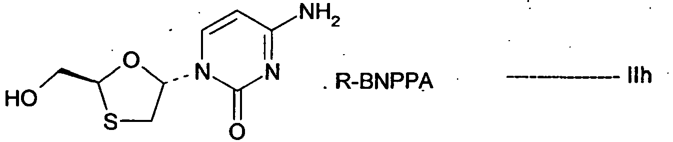

### DETAILED DESCRIPTION OF THE INVENTION

According to one aspect of the present invention, there is provided a process for resolution of ds(±)-4-amino-1-[2-(hydroxymethyl)-1,3-oxathiolan-5-yl]-2(1H)-pyrimidinone or trans(±)-4-amino-1-[2-(hydroxymethyl)-1,3-oxathiolan-5-yl]-2(1H)-pyrimidinone; or a mixture thereof which comprises:
a) reacting cis(±)-4-amino-1-[2-(hydroxymethyl)-1,3-oxathiolan-5-yl]-2(1H)-pyrimidinone or trans(±)-4-amino-1-[2-(hydroxymethyl)-1,3-oxathiolan-5-yl]-2(1 H)-pyrimidinone; or a mixture thereof with a chiral 1,1'-binaphtyl-2-2'-diyl hydrogen phosphate (BNPPA) to obtain corresponding one or more diastereomer compounds;
b) selectively crystallizing one diastereomer compound from water or water in combination with a solvent at a pH of 8.0 to 12.0; and
c) converting the separated diastereomer or diastereomers to the corresponding (2R-cis)-4-amino-1-[2-(hydroxymethyl)-1,3-oxathiolan-5-yl]-2(1H)-pyrimidinone, (2S-cis)-4-amino-1-[2-(hydroxymethyl)-1,3-oxathiolan-5-yl]-2(1H)-pyrimidinone, (2S-trans)-4-amino-1-[2-(hydroxymethyl)-1,3-oxathiolan-5-yl]-2(1H)-pyrimidinone or (2R-trans)-4-amino-1-[2-(hydroxymethyl)-1,3-oxathiolan-5-yl]-2(1H)-pyrimidinone.

Without bound to the nature of interaction (such as salt, complex etc.) between 4-amino-1-[2-(hydroxymethyl)-1,3-oxathiolan-5-yl]-2(1H)-pyrimidinone and chiral 1,1'-binaphtyl-2-2'-diyl hydrogen phosphate, the compounds of 4-amino-1-[2-(hydroxymethyl)-1,3-oxathiolan-5-yl]-2(1H)-pyrimidinone with 1,1'-binaphtyl-2-2'-diyl hydrogen phosphate are referred to, for example, as 4-amino-1-[2-(hydroxymethyl)-1,3-oxathiolan-5-yl]-2(1H)-pyrimidinone.1,1'-binaphtyl-2-2'-diyl hydrogen phosphate compounds. The reaction of cis(±)-4-amino-1-[2-(hydroxymethyl)-1,3-oxathiolan-5-yl]-2(1H)-pyrimidinone or trans(±)-4-amino-1-[2-(hydroxymethyl)-1,3-oxathiolan-5-yl]-2(1H)-pyrimidinone; or a mixture thereof with S-BNPPA or R-BNPPA in step (a) may preferably be carried out in water or in a solvent or a mixture thereof. Any suitable solvent such as an alcohol, ketone, acetonitril, N,N-dimethylformamide, tetrahydrofuran solvent may be used. Preferably, water or an alcohol or a mixture thereof may be used. Preferable alcohol is methanol or ethanol. Optionally, the pH of the reaction mass may be adjusted to, for example, above 8.0, preferably 8.5 to 12.0. The adjustment of the pH of the reaction mass may be carried out by using an acid or a base. There is no restriction on the use of a particular acid or a base, but preferably, inorganic bases such as alkaline metal hydroxides, carbonates or bicarbonates, or ammonia; or organic bases such as trimethylamine, triethylamine, mineral acids such as hydrochloric acid, sulfuric acid, phosphoric acid, organic acids such as acetic acid, formic acid, methane sulfonic acid may be used. The diastereomer compounds obtained in the step (a) may remain in the solution to proceed for further operations or may be isolated as a crystalline product or as a residual mass to proceed to the further operations. More than or less than one mole equivalent of chiral BNPPA with respect to the racemic 4-amino-1-[2-(hydroxymethyl)-1,3-oxathiolan-5-yl]-2(1H)-pyrimidinone may be used. If less than one mole equivalent of chiral BNPPA is used, one diastereomer compound may be formed in excess over the other diastereomer compound or only one diasteromer compound may be formed exclusively.

Selective crystallization may preferably be carried out in water or water in combination with a water miscible or water immiscible solvent. Any suitable solvent such as an alcohol, ketone, acetonitril, N,N-dimethylformamide, tetrahydrofuran solvent may be used. Preferably, water miscible solvent may be selected from methanol, ethanol and acetone and water immiscible solvent is selected from ethyl acetate, hexane and cyclohexane. Preferably, selective crystallization may be affected at a pH of 8.5 to 12.0. The adjustment of the pH of the reaction mass may be carried out by using an acid or a base. There is no restriction on the use of a particular acid or a base, but preferably, inorganic bases such as alkaline metal hydroxides, carbonates or bicarbonates, or ammonia; or organic bases such as trimethylamine, triethylamine, mineral acids such as hydrochloric acid, sulfuric acid, phosphoric acid, organic acids such as acetic acid, formic acid, methane sulfonic acid may be used.

The separated diastereomers is converted in step (c) to the corresponding enantiomer of 4-amino-1-[2-(hydroxymethyl)-1,3-oxathiolan-5-yl]-2(1H)-pyrimidinone preferably by suspending or dissolving the separated diasteromer in water or solvent or a mixture thereof and adding an acid or a base, preferably an acid. There is no restriction on the use of particular acid or base for the convertion. Alkaline metal hydroxides, carbonates or bicarbonates, or ammonia; or organic bases such as trimethylamine, triethylamine, mineral acids such as hydrochloric acid, sulfuric acid, phosphoric acid, organic acids such as acetic acid, formic acid, methane sulfonic acid may be mentioned for the conversion. Preferably, the conversion may be carried out in water, alcohols such as methanol, ethanol, isopropyl alcohol, ketones such as acetone, methyl ethyl ketone, methyl isopropyl ketone, methyl t-butyl ketone, acetonitril, N,N-dimethylformamide, tetrahydrofuran solvent may be used.

The compounds used as starting materials that is the cis(±)-4-amino-1-[2-(hydroxymethyl)-1,3-oxathiolan-5-yl]-2(1H)-pyrimidinone or trans(±)-4-amino-1-[2-(hydroxymethyl)-1,3-oxathiolan-5-yl]-2(1H)-pyrimidinone are known and can be obtained from known procedures as described in, for example, US 5,047,407.

The invention is not restricted to perfectly racemic compound and can also be applied to optical separation of enantiomeric mixture enantiomerically enriched with one enantiomer. Thus, the invention also serves as optical purification of 4-amino-1-[2-(hydroxymethyl)-1,3-oxathiolan-5-yl]-2(1H)-pyrimidinone isomers.

As a preferred process of the invention, the resolution of cis(±)-4-amino-1-[2-(hydroxymethyl)-1,3-oxathiolan-5-yl]-2(1H)-pyrimidinone may be carried out by the process which comprises:
a) reacting cis(±)-4-amino-1-[2-(hydroxymethyl)-1,3-oxathiolan-5-yl]-2(1H)-pyrimidinone with a chiral 1,1'-binaphtyl-2-2'-diyl hydrogenphosphate (BNPPA) to obtain corresponding one or more diastereomer compounds;
b) selectively crystallizing one diastereomer compound from water or water in combination with water miscible or immiscible solvent at a pH of 8.0 to 12.0; and
c) converting the separated distereomer or diastereomers to corresponding (2R-cis)-4-amino-1-[2-(hydroxymethyl)-1,3-oxathiolan-5-yl]-2(1H)-pyrimidinone or (2S-cis)-4-amino-1-[2-(hydroxymethyl)-1,3-oxathiolan-5-yl]-2(1H)-pyrimidinone.

As a specific example of the resolution of cis(±)-4-amino-1-[2-(hydroxymethyl)-1,3-oxathiolan-5-yl]-2(1H)-pyrimidinone to obtain (2R-cis)- 4-amino-1-[2-(hydroxymethyl)-1,3-oxathiolan-5-yl]-2(1H)-pyrimidinone may be represented by the following scheme:

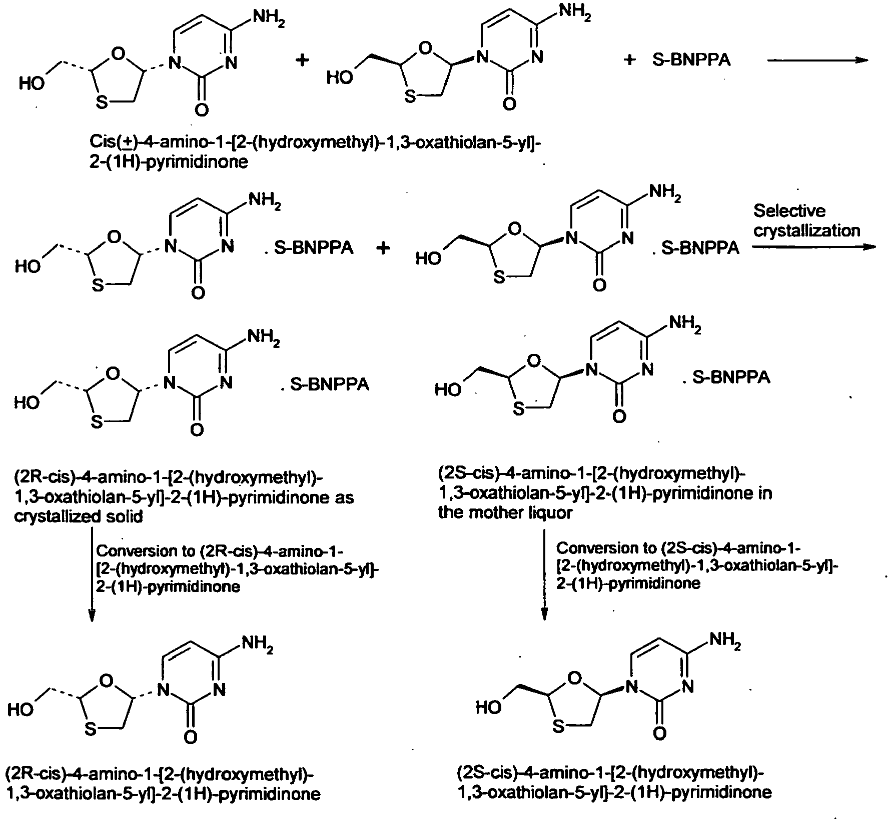

Similarly, R-BNPPA may be used instead of S-BNPPA to obtain 2S-cis-4-amino-1-[2-(hydroxymethyl)-1,3-oxathiolan-5-yl]-2(1H)-pyrimidinone from the crystallized compound and 2R-cis-4-amino-1-[2-(hydroxymethyl)-1,3-oxathiolan-5-yl]-2(1H)-pyrimidinone from the mother liquor.

The isomers of 4-amino-1-[2-(hydroxymethyl)-1,3-oxathiolan-5-yl]-2(1H)-pyrimidinone obtained by the process of the invention, may be converted into other isomer. Thus, for example, cis- or trans-isomers can be racemized by using an acid or a base, or by anomerization or transglycosylation as disclosed in US Patent No. 6,600,044.

According to another aspect of the present invention there is provided a compound selected from formulae IIa - IIh:

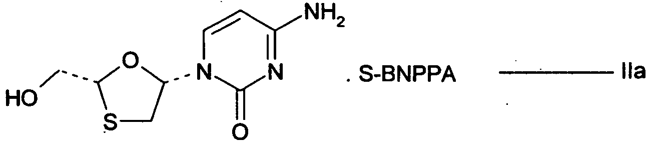

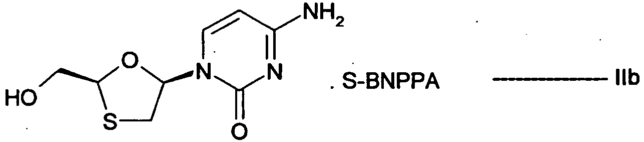

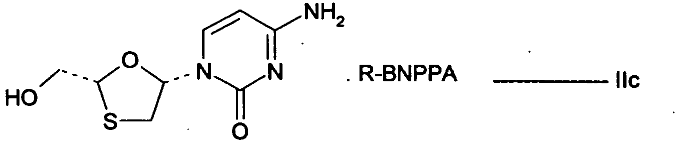

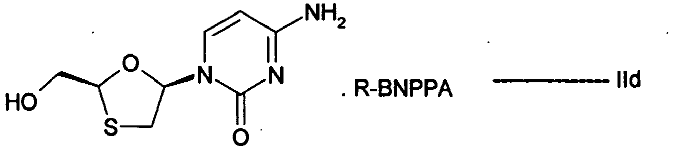

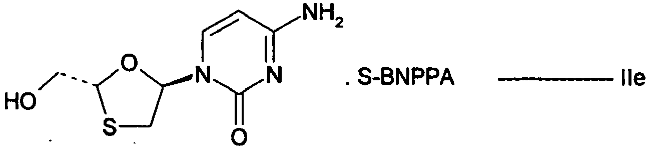

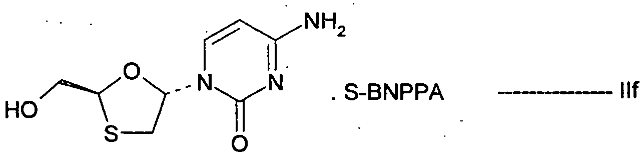

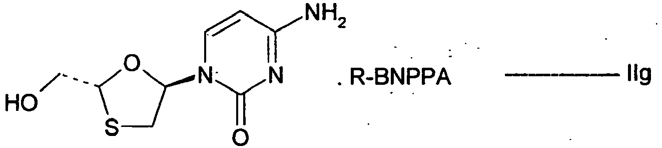

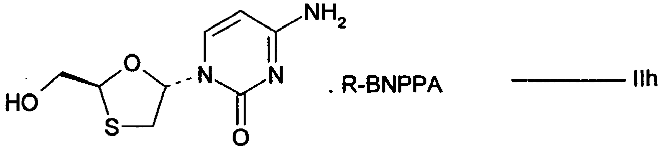

The invention will now be further described by the following examples, which are illustrative rather than limiting.

### Examples

### Example 1

### Preparation of (2R-cis)-4-Amino-1-[2-(hydroxymethyl)-1,3-oxathiolan-5-yl]-2(1H)-pyrimidinone.S-1,1'-binaphthyl-2,2'-diyl hydrogen phosphate:

38 g (0.109 moles) of S(+)-1,1'-binaphthyl-2,2'-diyl hydrogen phosphate was added to a solution of 25 gm (0.109 moles) of cis(±)-4-amino-1-[2-(hydroxymethyl)-1,3-oxathiolan-5-yl)-2(1H)-pyrimidinone in methanol (250 ml) at 60 - 65 deg C. The solution obtained was allowed to cool to room temperature and was stirred for 1 hour. The solid was filtered and dried under vacuum at 40 - 45 deg C for 2 hours.

The solid obtained above was taken in ethyl acetate (450 ml), water (225 ml), pH is adjusted to 11 by using aqueous sodium hydroxide solution (10%, 225 ml) and stirred the reaction mass for 1 hour. The solid was filtered and dried under vacuum at 40 - 45 deg C for 5 hours to obtain 20 gm of (2R-cis)-4-amino-1-[2-(hydroxymethyl)-1,3-oxathiolan-5-yl]-2(1H)-pyrimidinone.S-1.1'-binaphthyl-2,2'-diyl hydrogen phosphate (Chiral purity: 96%).

### Preparation of (2R-cis)-4-amino-1-[2-(hydroxymethyl)-1,3-oxathiolan-5-yl]-2(1H)-pyrimidinone (Lamivudine):

Aqueous hydrochloric acid (6N, 30 ml) was slowly added to a solution of 20 gm of the solid obtained above in water (200 ml) at 45 - 50 deg C. Stirred the reaction for 1 hour at room temperature. The solid S-1,1'-binaphthyl-2,2'-diyl hydrogen phosphate was filtered and the aqueous layer was neutralized with aqueous sodium hydroxide solution (30%, 20 ml). The solvent was recovered under vacuum at 40 - 45 deg C, the product obtained was dissolved in methanol (200 ml), filtered to remove the inorganic salts, the filtrate was concentrated under vacuum at 40 - 45 deg C and the residual solid obtained was dissolved in ethanol (50 ml), heated to 50 deg C, slowly allowed to room temperature, cooled to 10 deg C, filtered and dried at 40 - 45 deg C to obtain 5 gm of (2R-cis)-4-amino-1-[2-(hydroxymethyl)-1,3-oxathiolan-5-yl]-2(1H)-pyrimidinone (Chiral purity: 97.5%).

### Example 2

### Preparation of (2R-cis)-4-amino-1-[2-(hydroxymethyl)-1,3-oxathiolan-5-yl]-2(1H)-pyrimidinone.S-1,1'-binaphthyl-2,2'-diyl hydrogen phosphate:

38 gm (0.109 moles) of S(+)-1,1'-binaphthyl-2,2'-diyl hydrogen phosphate was added to a solution of 25 gm (0.109 moles) of cis(±)-4-amino-1-[2-(hydroxymethyl)-1,3-oxathiolan-5-yl]-2(1H)-pyrimidinone in methanol (250 ml) at 60 - 65 deg C. The solution obtained was allowed to cool to room temperature and was stirred for 1 hour. The solid was filtered and dried under vacuum at 40-45 deg C for 2 hours.

The solid obtained above was taken in water (500 ml), pH was adjusted to 11 by using aqueous sodium hydroxide solution (10%, 250ml) and stirred the reaction mass for 1 hour. The solid was filtered and dried under vacuum at 40 - 45 deg C for 6 hours to obtain 25 gm of (2R-cis)-4-amino-1-[2-(hydroxymethyl)-1,3-oxathiolan-5-yl]-2(1H)-pyrimidinone.S-1,1'-binaphthyl-2,2'-diyl hydrogen phosphate (Chiral purity: 97%).

### Preparation of (2R-cis)-4-amino-1-[2-(hydroxymethyl)-1,3-oxathiolan-5-yl]-2(1H)-pyrimidinone (Lamivudine):

Aqueous hydrochloric acid (6N, 25 ml) was slowly added to a solution of 25 g of (2R-cis)-4-amino-1-[2-(hydroxymethyl)-1,3-oxathiolan-5-yl]-2(1H)-pyrimidinone.S-1,1'-binaphthyl-2,2'-diyl hydrogen phosphate in methanol (250 ml). Stirred the mass for 1 hour at room temperature. The solid was collected and the residual solid obtained was dissolved in ethanol (50 ml), heated to 50 deg C, slowly allowed to room temperature, cooled to 10 deg C, filtered and dried at 40 - 45 deg C to obtain 7 gm of (2R-cis)-4-amino-1-[2-(hydroxymethyl)-1,3-oxathiolan-5-yl]-2(1H)-pyrimidinone (Chiral purity: 99.85%).

### Example 3

### Preparation of (2R-cis)-4-amino-1-[2-(hydroxymethyl)-1,3-oxathiolan-5-yl]-2(1H)-pyrimidinone.S-1,1'-binaphthyl-2,2'-diyl hydrogen phosphate:

Aqueous sodium hydroxide solution (10%, 125 ml), 38 gm (0.109 moles) of S(+)-1,1'-binaphthyl-2,2'-diyl hydrogen phosphate were added to a solution of 25 gm (0.109 moles) of cis(±)-4-amino-1-[2-(hydroxymethyl)-1,3-oxathiolan-5-yl]-2(1H)-pyrimidinone in water (500 ml) at 40 - 45 deg C. The solution obtained was allowed to cool to room temperature and was stirred for 1 hour. The solid was collected and dried under vacuum at 40 - 45 deg C four 5 hours to obtain 20 gm of (2R-cis)-4-amino-1-[2-(hydroxymethyl)-1,3-oxathiolan-5-yl]-2(1H)-pyrimidinone.S-1,1'-binaphthyl-2,2'-diyl hydrogen phosphate (Chiral purity: 99%).

### Preparation of (2R-cis)-4-amino-1-[2-(hydroxymethyl)-1,3-oxathiolan-5-yl]-2(1H)-pyrimidinone (Lamivudine):

Aqueous hydrochloric acid (6N, 20 ml) was slowly added to a solution of 20 gm of the solid obtained above in ethanol (200 ml). Stirred the mass for 1 hour at room temperature. The solid was collected and was dissolved in a mixture of ethanol and water (30 ml, 1: 3 by volume), heated to 50 deg C and the pH was adjusted to 7.5 with caustic soda lye (2 ml) at 50 deg C, slowly allowed to room temperature, cooled to 5 deg C, filtered and dried at 40 - 45 deg C to obtain 8 gm of (2R-cis)-4-amino-1-[2-(hydroxymethyl)-1,3-oxathiolan-5-yl]-2(1H)-pyrimidinone (Chiral purity: 99.8%)

### Example 4

### Preparation of (2R-cis)-4-amino-1-[2-(hydroxymethyl)-1,3-oxathiolan-5-yl]-2(1H)-pyrimidinone.S-1,1'-binaphthyl-2,2'-diyl hydrogen phosphate:

Aqueous sodium hydroxide solution (10%, 125 ml), 19 gm (0.054 moles) of S(+)-1,1'-binaphthyl-2,2'-diyl hydrogen phosphate were added to a solution of 25 gm (0.109 moles) of cis(±)-4-amino-1-[2-(hydroxymethyl)-1,3-oxathiolan-5-yl]-2(1H)-pyrimidinone in water (500 ml) at 40 - 45 deg C. The solution obtained was allowed to cool to room temperature and was stirred for 1 hour. Cooled the mass to 20 deg C, ethyl acetate (125 ml) was added and stirred for 15 minutes. The solid was collected and dried under vacuum at 40 - 45 deg C for 5 hours to obtain 25 gm of (2R-cis)-4-amino-1-[2-(hydroxymethyl)-1,3-oxathiolan-5-yl]-2(1H)-pyrimidinone.S-1,1'-binaphthyl-2,2'-diyl hydrogen phosphate (Chiral purity: 99.6%).

### Preparation of (2R-cis)-4-amino-1-[2-(hydroxymethyl)-1,3-oxathiolan-5-yl]-2(1H)-pyrimidinone (Lamivudine):

Aqueous hydrochloric acid (6N, 25 ml) was added drop wise to a solution of 25 gm of the solid obtained above in ethanol (250 ml), stirred the mass for 1 hour at room temperature and cooled the mass to 20 deg C. The solid was collected and was dissolved in a mixture of ethanol and water (38 ml, 1: 3 by volume), heated to 50 deg C and the pH was adjusted to 7.5 with caustic soda lye (2.5 ml) at 50 deg C, slowly allowed to room temperature, cooled to 5 deg C, filtered and dried at 40 - 45 deg C to obtain 10 gm of (2R-cis)-4-amino-1-[2-(hydroxymethyl)-1,3-oxathiolan-5-yl]-2(1H)-pyrimidinone (Chiral purity: 100%)

### Example 5

### Preparation of (2S-cis)-4-amino-1-[2-(hydroxymethyl)-1,3-oxathiolan-5-yl]-2(1H)-pyrimidinone.R-1,1'-binaphthyl-2,2'-diyl hydrogen phosphate:

Aqueous sodium hydroxide solution (10%, 125 ml), 38 gm (0.109moles) of R(-)-1,1'-binaphthyl-2,2'-diyl hydrogen phosphate were added to a solution of 25 gm (0.109 moles) of cis(±)-4-amino-1-[2-(hydroxymethyl)-1,3-oxathiolan-5-yl]-2(1 H)-pyrimidinone in water (500 ml) at 40 - 45 deg C. The solution obtained was allowed to cool to 20 deg C and was stirred for 1 hour. Cooled the mass to 10 deg C, ethyl acetate (125 ml) was added and stirred for 15 minutes. The solid was collected and the filtrate was kept for further processing. The solid was dried under vacuum at 40 - 45 deg C to obtain 25 gm of (2S-cis)-4-amino-1-[2-(hydroxymethyl)-1,3-oxathiolan-5-yl]-2(1H)-pyrimidinone.R-1,1'-binaphthyl-2,2'-diyl hydrogen phosphate (Chiral purity: 97.1 %).

### Preparation of (2S-cis)-4-amino-1-[2-(hydroxymethyl)-1,3-oxathiolan-5-yl]-2(1H)-pyrimidinone:

Aqueous hydrochloric acid (6N, 25 ml) was added drop wise to a solution of 25 gm of the solid obtained above in ethanol (250 ml), stirred the mass for 1 hour at room temperature and cooled the mass to 20 deg C. The solid was collected and was dissolved in a mixture of ethanol and water (38 ml, 1: 3 by volume), heated to 50 deg C and the pH was adjusted to 7.5 with caustic soda lye (2.5 ml) at 50 deg C, slowly allowed to room temperature, cooled to 5 deg C, filtered and dried at 40 - 45 deg C to obtain 10 gm of (2S-cis)-4-amino-1-[2-(hydroxymethyl)-1,3-oxathiolan-5-yl]-2(1H)-pyrimidinone (Chiral purity: 99.8%).

### Preparation of (2R-cis)-4-amino-1-[2-(hydroxymethyl)-1,3-oxathiolan-5-yl]-2(1H)-pyrimidinone (Lamivudine) from the filtrate:

The filtrate obtained from the step of preparation of (2S-cis)-4-amino-1-[2-(hydroxymethyl)-1,3-oxathiolan-5-yl]-2(1H)-pyrimidinone.R-1,1'-binaphthyl-2,2'-diyl hydrogen phosphate was concentrated and the residual solid obtained was dissolved in the mixture of ethanol (250 ml) and aqueous hydrochloric acid (6N, 25 ml ), stirred the mass for 1 hour at room temperature and cooled the mass to 20 deg C. The solid was collected and was dissolved in a mixture of ethanol and water (38 ml, 1: 3 by volume), heated to 50 deg C and the pH was adjusted to 7.5 with caustic soda lye (2.5 ml) at 50 deg C, slowly allowed to room temperature, cooled to 5 deg C, filtered and dried at 40 - 45 deg C to obtain 10 gm of (2R-cis)-4-amino-1-[2-(hydroxymethyl)-1,3-oxathiolan-5-yl]-2(1H)-pyrimidinone (Chiral purity: 99.7%).

### Example 6

### Preparation of trans(-)-4-amino-1-[2-(hydroxymethyl)-1,3-oxathiolan-5-yl]-2(H)-pyrimidinone.S-1,1'-binaphthyl-2,2'-diyl hydrogen phosphate:

Aqueous sodium hydroxide solution (10%, 120 ml), 38 gm (0.109 moles) of S(+)-1,1'-binaphthyl-2,2'-diyl hydrogen phosphate were added to a solution of 25 gm (0.109.moles) of trans(±)-4-amino-1-[2-(hydroxymethyl)-1,3-oxathiolan-5-yl]-2(1 H)-pyrimidinone in water (500 ml) at 40 - 45 deg C. The solution obtained was allowed to cool to room temperature and was stirred for 1 hour. The solid was collected and dried under vacuum at 40 - 45 deg C for 5 hours to obtain 20 gm of trans(-)-4-amino-1-[2-(hydroxymethyl)-1,3-oxathiolan-5-yl]-2(1H)-pyrimidinone.S-1,1'-binaphthyl-2,2'-diyl hydrogen phosphate (Chiral purity: 99%).

### Preparation of trans(-)-4-amino-1-[2-(hydroxymethyl)-1,3-oxathiolan-5-yl]-2(1H)-pyrimidinone:

Aqueous hydrochloric acid (6N, 20 ml) was slowly added to a solution of 20 gm of the solid obtained above in ethanol (200 ml). Stirred the mass for 1 hour at room temperature. The solid was collected and was dissolved in a mixture of ethanol and water (30 ml, 1: 3 by volume), heated to 50 deg C and the pH was adjusted to 7.5 with caustic soda lye (2 ml) at 50 deg C, slowly allowed to room temperature, cooled to 5 deg C, filtered and dried at 40 - 45 deg C to obtain 8 gm of trans(-)-4-amino-1-[2-(hydroxymethyl)-1,3-oxathiolan-5-yl]-2(1 H)-pyrimidinone (Chiral purity: 99.3%).

### Example 7

The example 4 was repeated but with 25 gm of the mixture of cis(±)- and trans(±)-4-amino-1-[2-(hydroxymethyl)-1,3-oxathiolan-5-yl]-2(1H)-pyrimidinone (1:1) instead of cis(±)-4-amino-1-[2-(hydroxymethyl)-1,3-oxathiolan-5-yl]-2(1H)-pyrimidinone to obtain 4.8 gm of (2R-cis)-4-amino-1-[2-(hydroxymethyl)-1,3-oxathiolan-5-yl]-2(1 H)-pyrimidinone with chiral purity 97.3%.

## Claims

1. A process for resolution of cis(±)-4-amino-1-[2-(hydroxymethyl)-1,3-oxathiolan-5-yl]-2(1H)-pyrimidinone or trans(±)-4-amino-1-[2-(hydroxymethyl)-1,3-oxathiolan-5-yl]-2(1H)-pyrimidinone; or a mixture thereof which comprises:
a) reacting cis(±)-4-amino-1-[2-(hydroxymethyl)-1,3-oxathiolan-5-yl]-2(1H)-pyrimidinone or trans(±)-4-amino-1-[2-(hydroxymethyl)-1,3-oxathiolan-5-yl]-2(1H)-pyrimidinone; or a mixture thereof with a chiral 1,1'-binaphthyl-2-2'-diyl hydrogenphosphate (BNPPA) to obtain corresponding one or more diastereomer compounds;
b) selectively crystallizing one diastereomer compound from water or water in combination with a solvent at a pH of 8.0 to 12.0; and
c) converting the separated diastereomer or diastereomers to the corresponding (2R-cis)-4-amino-1-[2-(hydroxymethyl)-1,3-oxathiolan-5-yl]-2(1 H)-pyrimidinone, (2S-cis)-4-amino-1-[2-(hydroxymethyl)-1,3-oxathiolan-5-yl]-2(1H)-pyrimidinone, (2S-trans)-4-amino-1-[2-(hydroxymethyl)-1,3-oxathiolan-5-yl]-2(1H)-pyrimidinone or (2R-trans)-4-amino-1-[2-(hydroxymethyl)-1,3-oxathiolan-5-yl]-2(1H)-pyrimidinone.

2. The process as claimed in claim 1, wherein the step (a) process is carried out in water or in a solvent or a mixture thereof.

3. The process as claimed in claim 2, wherein the solvent used in the process is selected from an alcohol, a ketone, acetonitrile, N,N-dimethylformamide and tetrahydrofuran.

4. The process as claimed in claim 3, wherein the alcohol used in the process is selected from methanol and ethanol.

5. The process as claimed in claim 1, wherein the reaction in step (a) is carried out at pH of 8.0 to 12.0.

6. The process as claimed in claim 1, wherein the pH of the reaction mass is adjusted with a base or acid in step (a).

7. The process as claimed in claim 6, wherein the base is inorganic base or organic base.

8. The process as claimed in claim 7, wherein the inorganic base is alkaline metal hydroxides, carbonates or bicarbonates and ammonia.

9. The process as claimed in claim 7, wherein the organic base is trimethylamine and triethylamine.

10. The process as claimed in claim 1, wherein the crystallization in step (b) is carried out in water or water in combination with a water miscible or water immiscible solvent.

11. The process as claimed in claim 1, wherein the reaction in step (b) is carried out at pH of 8.5 to 12.0.

12. The process as claimed in claim 1, wherein the pH of the reaction mass is adjusted with a base or acid in step (b).

13. The process as claimed in claim 11, wherein the base is inorganic base or organic base.

14. The process as claimed in claim 13, wherein the inorganic base is alkaline metal hydroxides, carbonates or bicarbonates and ammonia.

15. The process as claimed in claim 13, wherein the organic base is trimethylamine and triethylamine.

16. The process as claimed in claim 1, wherein the reaction in step (c) conversion is carried out in base or acid.

17. The process as claimed in claim 16, wherein the acid is a mineral acid or an organic acid.

18. The process as claimed in claim 17, wherein the mineral acid is hydrochloric acid, sulfuric acid and phosphoric acid.

19. The process as claimed in claim 17, wherein the organic acid is acetic acid, formic acid and methane sulfonic acid.

20. A compound selected from the formula IIa to IIh:

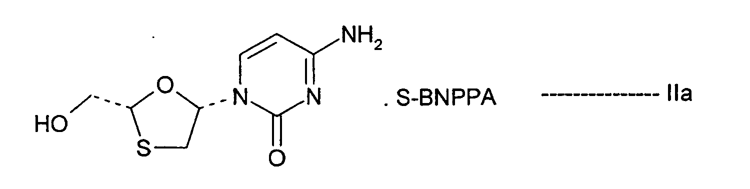

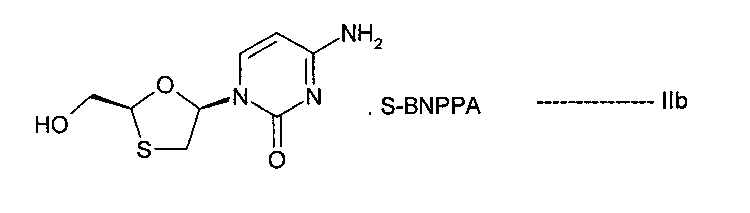

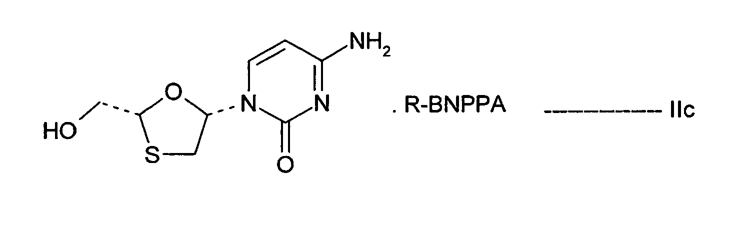

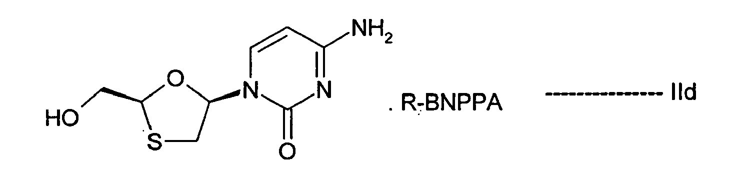

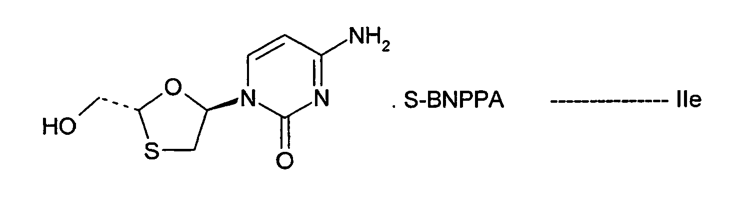

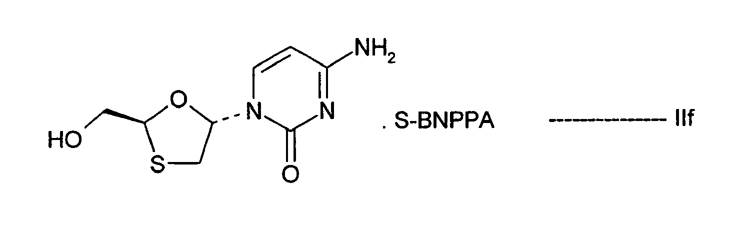

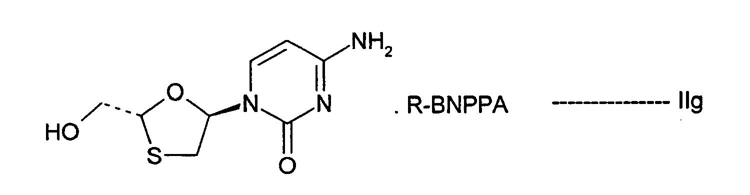

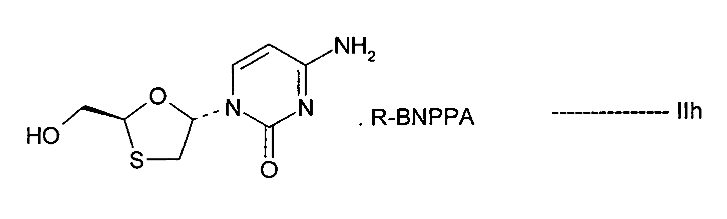

## Patentansprüche

1. Verfahren zur Aufspaltung von cis(±)-4-Amino-1-[2-(hydroxymethyl)-1,3-oxathiolan-5-yl]-2(1H)-pyrimidinon oder trans(±)-4-Amino-1-[2-(hydroxymethyl)-1,3-oxathiolan-5-yl]-2(1H)-pyrimidinon oder einer Mischung davon, wobei das Verfahren Folgendes umfasst:
a) Umsetzen von cis(±)-4-Amino-1-[2-(hydroxymethyl)-1,3-oxathiolan-5-yl]-2(1H)-pyrimidinon oder trans(±)-4-Amino-1-[2-(hydroxymethyl)-1,3-oxathiolan-5-yl]-2(1H)-pyrimidinon oder einer Mischung davon mit einem chiralen 1,1'-Binaphthyl-2,2'-diylhydrogenphosphat (BNPPA), um eine oder mehrere entsprechende Diastereomerverbindungen zu erhalten;
b) selektives Umkristallisieren einer Diastereomerverbindung aus Wasser oder Wasser in Kombination mit einem Lösungsmittel bei einem pH-Wert von 8,0 bis 12,0 und
c) Umwandeln des getrennten Diastereomers oder der getrennten Diastereomere in das entsprechende (2R-cis)-4-Amino-1-[2-(hydroxymethyl)-1,3-oxathiolan-5-yl]-2(1H)-pyrimidinon, (2S-cis)-4-Amino-1-[2-(hydroxymethyl)-1,3-oxathiolan-5-yl]-2(1H)-pyrimidinon, (2S-trans)-4-Amino-1-[2-(hydroxymethyl)-1,3-oxathiolan-5-yl]-2(1H)-pyrimidinon oder (2R-trans)-4-Amino-1-[2-(hydroxymethyl)-1,3-oxathiolan-5-yl]-2(1H)-pyrimidinon.

2. Verfahren nach Anspruch 1, wobei das Verfahren von Schritt (a) in Wasser oder in einem Lösungsmittel oder einer Mischung davon ausgeführt wird.

3. Verfahren nach Anspruch 2, wobei das in dem Verfahren verwendete Lösungsmittel aus einem Alkohol, einem Keton, Acetonitril, N,N-Dimethylformamid und Tetrahydrofuran ausgewählt ist.

4. Verfahren nach Anspruch 3, wobei der in dem Verfahren verwendete Alkohol aus Methanol und Ethanol ausgewählt ist.

5. Verfahren nach Anspruch 1, wobei die Reaktion in Schritt (a) bei einem pH-Wert von 8,0 bis 12,0 ausgeführt wird.

6. Verfahren nach Anspruch 1, wobei der pH-Wert der Reaktionsmasse in Schritt (a) mit einer Base oder Säure eingestellt wird.

7. Verfahren nach Anspruch 6, wobei es sich bei der Base um eine anorganische Base oder organische Base handelt.

8. Verfahren nach Anspruch 7, wobei es sich bei der anorganischen Base um Alkalimetallhydroxide, - carbonate oder -bicarbonate und Ammoniak handelt.

9. Verfahren nach Anspruch 7, wobei es sich bei der organischen Base um Trimethylamin und Triethylamin handelt.

10. Verfahren nach Anspruch 1, wobei die Umkristallisation in Schritt (b) in Wasser oder Wasser in Kombination mit einem mit Wasser mischbaren oder mit Wasser unmischbaren Lösungsmittel ausgeführt wird.

11. Verfahren nach Anspruch 1, wobei die Reaktion in Schritt (b) bei einem pH-Wert von 8,5 bis 12,0 ausgeführt wird.

12. Verfahren nach Anspruch 1, wobei der pH-Wert der Reaktionsmasse in Schritt (b) mit einer Base oder Säure eingestellt wird.

13. Verfahren nach Anspruch 11, wobei es sich bei der Base um eine anorganische Base oder organische Base handelt.

14. Verfahren nach Anspruch 13, wobei es sich bei der anorganischen Base um Alkalimetallhydroxide, - carbonate oder -bicarbonate und Ammoniak handelt.

15. Verfahren nach Anspruch 13, wobei es sich bei der organischen Base um Trimethylamin und Triethylamin handelt.

16. Verfahren nach Anspruch 1, wobei die Reaktion in der Umwandlung von Schritt (c) in einer Base oder Säure ausgeführt wird.

17. Verfahren nach Anspruch 16, wobei es sich bei der Säure um eine Mineralsäure oder eine organische Säure handelt.

18. Verfahren nach Anspruch 17, wobei es sich bei der Mineralsäure um Salzsäure, Schwefelsäure und Phosphorsäure handelt.

19. Verfahren nach Anspruch 17, wobei es sich bei der organischen Säure um Essigsäure, Ameisensäure und Methansulfonsäure handelt.

20. Verbindung, die aus den Formeln IIa bis IIh ausgewählt ist:

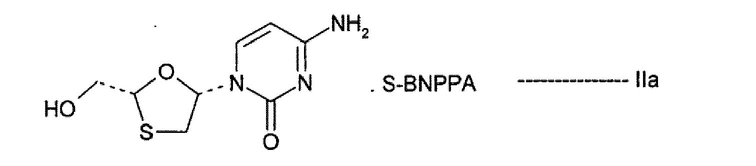

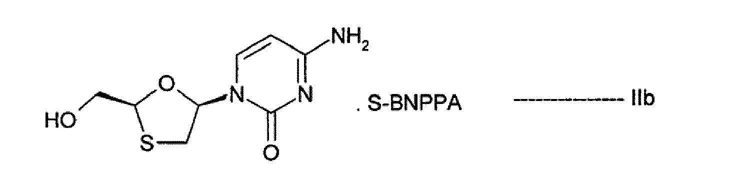

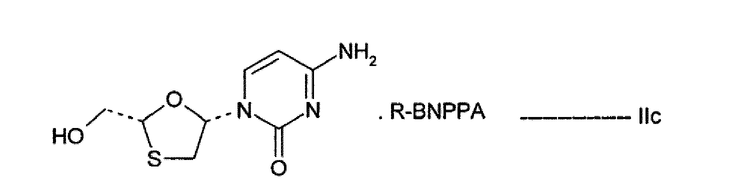

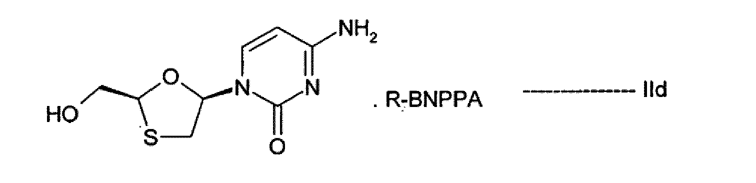

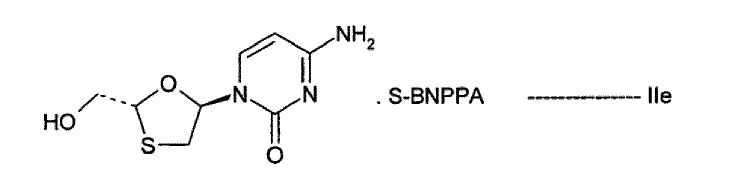

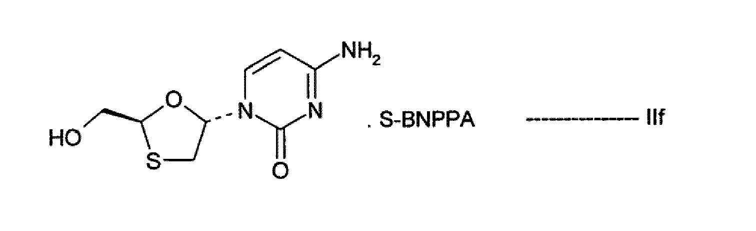

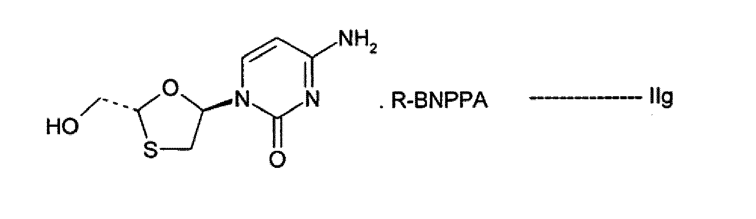

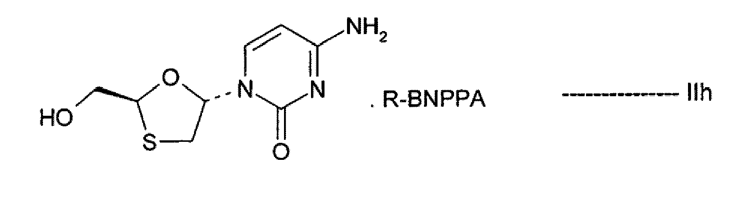

## Revendications

1. Procédé pour la résolution de *cis*-(*±*)-4-amino-1-[2-(hydroxyméthyl)-1,3-oxathiolan-5-yl]-2(1*H*)-pyrimidinone ou de *trans*-(*±*)-4-amino-1-[2-(hydroxyméthyl)-1,3-oxathiolan-5-yl]-2(1*H*)-pyrimidinone ou d'un mélange de celles-ci qui comprend :
a) la réaction de *cis*-(*±*)-4-amino-1-[2-(hydroxyméthyl)-1,3-oxathiolan-5-yl]-2(1*H*)-pyrimidinone ou de *trans*-(*±*)-4-amino-1-[2-(hydroxyméthyl)-1,3-oxathiolan-5-yl]-2(1*H*)-pyrimidinone ou d'un mélange de celles-ci avec un hydrogénophosphate de 1,1'-binaphtyl-2,2'-diyle (BNPPA) chiral pour obtenir un ou plusieurs composés diastéréoisomères correspondants ;
b) la cristallisation sélective d'un composé diastéréoisomère dans de l'eau ou de l'eau en association avec un solvant à un pH de 8,0 à 12,0 ; et
c) la conversion du ou des diastéréoisomères séparés en la (2R-cis)-4-amino-1-[2-(hydroxyméthyl)-1,3-oxathiolan-5-yl]-2(1*H*)-pyrimidinone, (*2S-cis*)-4-amino-1-[2-(hydroxyméthyl)-1,3-oxathiolan-5-yl]-2(1*H*)-pyrimidinone, (2*S*-*trans*)-4-amino-1-[2-(hydroxyméthyl)-1,3-oxathiolan-5-yl]-2(1*H*)-pyrimidinone ou (2*R*-*trans*)-4-amino-1-[2-(hydroxyméthyl)-1,3-oxathiolan-5-yl]-2(1*H*)-pyrimidinone correspondante.

2. Procédé selon la revendication 1, dans lequel le procédé de l'étape (a) est effectué dans de l'eau ou dans un solvant ou un mélange de ceux-ci.

3. Procédé selon la revendication 2, dans lequel le solvant utilisé dans le procédé est choisi parmi un alcool, une cétone, l'acétonitrile, le *N,N-*diméthylformamide et le tétrahydrofurane.

4. Procédé selon la revendication 3, dans lequel l'alcool utilisé dans le procédé est choisi entre le méthanol et l'éthanol.

5. Procédé selon la revendication 1, dans lequel la réaction dans l'étape (a) est effectuée à un pH de 8,0 à 12,0.

6. Procédé selon la revendication 1, dans lequel le pH de la masse réactionnelle est ajusté avec une base ou un acide dans l'étape (a).

7. Procédé selon la revendication 6, dans lequel la base est une base inorganique ou une base organique.

8. Procédé selon la revendication 7, dans lequel la base inorganique est choisie parmi les hydroxydes, carbonates ou bicarbonates de métaux alcalins et l'ammoniac.

9. Procédé selon la revendication 7, dans lequel la base organique est choisie entre la triméthylamine et la triéthylamine.

10. Procédé selon la revendication 1, dans lequel la cristallisation dans l'étape (b) est effectuée dans de l'eau ou de l'eau en association avec un solvant miscible avec l'eau ou non miscible avec l'eau.

11. Procédé selon la revendication 1, dans lequel la réaction dans l'étape (b) est effectuée à un pH de 8,5 à 12,0.

12. Procédé selon la revendication 1, dans lequel le pH de la masse réactionnelle est ajusté avec une base ou un acide dans l'étape (b).

13. Procédé selon la revendication 11, dans lequel la base est une base inorganique ou une base organique.

14. Procédé selon la revendication 13, dans lequel la base inorganique est choisie parmi les hydroxydes, carbonates ou bicarbonates de métaux alcalins et l'ammoniac.

15. Procédé selon la revendication 13, dans lequel la base organique est choisie entre la triméthylamine et la triéthylamine.

16. Procédé selon la revendication 1, dans lequel la réaction dans la conversion de l'étape (c) est effectuée dans une base ou un acide.

17. Procédé selon la revendication 16, dans lequel l'acide est un acide minéral ou un acide organique.

18. Procédé selon la revendication 17, dans lequel l'acide minéral est choisi parmi l'acide chlorhydrique, l'acide sulfurique et l'acide phosphorique.

19. Procédé selon la revendication 17, dans lequel l'acide organique est choisi parmi l'acide acétique, l'acide formique et l'acide méthanesulfonique.

20. Composé choisi parmi les formules IIa à IIh :
